# FASCICULE DE BREVET EUROPEEN

(11) **EP 2 170 443 B1**
(45) Date de publication et mention de la délivrance du brevet: **04.09.2019**
(21) Numéro de dépôt: 08806146.0
(22) Date de dépôt: 01.07.2008
(51) Int. Cl.: A61M 15/00

(54) **DISPOSITIF D'INHALATION COMPORTANT UN DISPOSITIF D'OUVERTURE DE RESERVOIR DE PRODUIT FLUIDE**
INHALATIONSVORRICHTUNG MIT EINER ÖFFNUNGSVORRICHTUNG ZUR ÖFFNUNG EINES FLUIDPRODUKTRESERVOIRS
INHALATION DEVICE WITH AN OPENING DEVICE FOR OPENING A FLUID PRODUCT RESERVOIR

(30) Priorité: 03.07.2007 FR 0756237
(43) Date de publication de la demande: 07.04.2010
(73) Titulaire: Aptar France SAS, 27110 Le Neubourg (FR)
(72) Inventeur: CHOPARD, David, F-27490 Autheuil Authouillet (FR)
(74) Mandataire: CAPRI
(86) Numéro de dépôt international: PCT/FR2008/051222
(87) Numéro de publication internationale: WO 2009/007640

(56) Documents cités:
- WO-A-2005/037353
- WO-A-2006/062651
- WO-A-2006/079750
- DE-A1- 19 757 208
- GB-A- 2 420 982
- US-A1- 2006 048 780

## Description

La présente invention concerne un dispositif d'ouverture de réservoir et un dispositif de distribution de produit fluide, plus particulièrement un inhalateur de poudre sèche, comportant un tel dispositif d'ouverture.

Les inhalateurs de poudre sèche sont bien connus dans l'état de la technique. Il en existe différentes sortes. Un premier type d'inhalateur contient un réservoir recevant une multitude de doses de poudre, l'inhalateur étant pourvu de moyens de dosage permettant à chaque actionnement de séparer une dose de cette poudre du réservoir pour l'amener dans un conduit d'expulsion afin d'être distribué à l'utilisateur. Un autre type d'inhalateur consiste à emballer les doses de poudre dans des réservoirs individuels prédosés, puis d'ouvrir un de ces réservoirs à chaque actionnement de l'inhalateur. Cette mise en oeuvre assure une meilleure étanchéité de la poudre, puisque chaque dose n'est ouverte qu'au moment de son expulsion. Pour réaliser ces réservoirs individuels, diverses variantes ont déjà été proposées, telle qu'une bande de blister allongé ou des blisters disposés sur un disque circulaire rotatif. Des inhalateurs comportant des réservoirs individuels, tels que des capsules, qui sont à charger dans l'inhalateur juste avant l'utilisation de celui-ci ont également été décrits dans l'état de la technique. L'avantage de ces dispositifs est qu'il n'est pas nécessaire de stocker l'ensemble des doses à l'intérieur de l'appareil, de sorte que celui-ci peut être de dimension réduite. Par contre évidemment, l'utilisation est plus complexe, puisque l'utilisateur est obligé de charger une capsule dans l'inhalateur avant chaque utilisation. Tous les types d'inhalateurs décrits ci-dessus et existants présentent des avantages et des inconvénients liés à leur structure et à leur fonctionnement. Ainsi, avec certains inhalateurs, se pose le problème de la précision et de la reproductibilité du dosage à chaque actionnement. De même, l'efficacité de la distribution, c'est-à-dire la partie de la dose qui pénètre effectivement dans les poumons de l'utilisateur pour avoir un effet thérapeutique bénéfique, est également un problème qui se présente avec un certain nombre d'inhalateurs. Une solution pour résoudre ce problème spécifique a été de synchroniser l'expulsion de la dose avec l'inhalation du patient. A nouveau, ceci pouvait générer des inconvénients, à savoir que généralement dans ce type de dispositif, la dose est chargée dans un conduit d'expulsion avant l'inhalation, puis l'expulsion est synchronisée avec l'inhalation. Ceci signifie que si l'utilisateur laisse tomber, secoue ou manipule de manière non souhaitée ou inadaptée l'inhalateur entre le moment où il a changé la dose (soit à partir d'un réservoir multidoses soit à partir d'un réservoir individuel) et au moment où il inhale, il risque de perdre toute ou partie de cette dose, celle-ci pouvant se répartir à l'intérieur de l'appareil. Dans ce cas il peut se présenter un gros risque de surdosage lors de la prochaine utilisation du dispositif. L'utilisateur qui se rendra compte que sa dose n'est pas complète chargera une nouvelle dose dans l'appareil, et lors de l'inhalation de cette nouvelle dose, une partie de la dose précédente perdue dans l'appareil pourrait être alors expulsée en même temps que la nouvelle dose, provoquant un surdosage. Selon les traitements envisagés, ce surdosage peut être très néfaste et c'est une exigence de plus en plus forte des autorités de tous les pays de limiter au maximum ce risque de surdosage. Concernant l'ouverture des réservoirs individuels, il a été proposé de peler ou décoller la couche de fermeture. Ceci présente l'inconvénient d'une maîtrise difficile des forces à appliquer pour garantir une ouverture totale sans risquer d'ouvrir le réservoir suivant, particulièrement si les moyens d'ouverture doivent être actionnés par l'inhalation. En variante, il a été proposé de percer la paroi ou couche de fermeture. Ceci peut présenter l'inconvénient que des parties de paroi découpées risquent de retenir une partie de la dose à l'intérieur du réservoir, la précision et reproductibilité de dosage n'étant alors pas garanties. Un autre problème avec le perçage est de garantir un écoulement d'inhalation suffisant pour sortir la poudre du blister percé et la transporter vers l'orifice de distribution. Les documents WO 2006/062651, US 2006/048780, WO 2006/079750, WO 2005/037353 et DE 19757208 décrivent des dispositifs à perçage de l'état de la technique.

La présente invention a pour but de fournir un dispositif d'ouverture de réservoir et un dispositif de distribution de produit fluide, en particulier un inhalateur de poudre sèche, qui ne reproduisent pas les inconvénients susmentionnés.

En particulier, la présente invention a pour but de fournir de tels dispositifs qui soient simples et peu coûteux à fabriquer et à assembler, fiables d'utilisation, garantissant une précision de dosage et une reproductibilité du dosage à chaque actionnement, fournissant un rendement optimal quant à l'efficacité du traitement, en permettant de distribuer une part importante de la dose au niveau des zones à traiter, en particulier les poumons, évitant de manière sûre et efficace les risques des surdosages, de dimensions aussi petites que possibles, tout en garantissant une étanchéité et une intégrité absolue de toutes les doses jusqu'à leur expulsion.

La présente invention a aussi pour but de fournir de tels dispositifs pour lesquels la variabilité de la dose émise est réduite.

La présente invention a donc pour objet un dispositif d'inhalation tel que décrit dans la revendication 1. Des modes de réalisation avantageux sont décrits dans les revendications dépendantes.

Ces caractéristiques et avantages et d'autres de la présente invention apparaîtront plus clairement au cours de la description détaillée suivante de plusieurs modes et variantes de réalisation de celle-ci, faite en référence aux dessins joints, donnés à titres d'exemples non limitatifs, sur lesquels,
- la figure 1 est une vue schématique en perspective d'un élément de perçage selon un mode de réalisation de la présente invention,
- la figure 2 est une vue en section transversale de l'élément de perçage de la figure 1,
- la figure 3 est une vue en section transversale d'une partie d'un inhalateur comportant un élément de perçage tel que représenté sur les figures 1 et 2, et
- les figures 4 à 6 sont des vues schématiques de variantes de réalisation d'un élément de perçage.

En référence aux figures, l'invention concerne principalement un dispositif d'ouverture de réservoirs individuels pour un inhalateur de poudre sèche. Elle s'applique en particulier à des inhalateurs dans lesquels les réservoirs 20 sont formés par des blisters disposés les uns derrière les autres sur une bande souple (non représentée). Chaque réservoir ou blister 20 comporte une paroi de fermeture étanche 21 et contient une dose de poudre. Avantageusement, cette bande se déroule progressivement à chaque actionnement, une roue d'indexage (non représentée) étant prévue pour faire avancer la bande et pour recevoir un blister à ouvrir à chaque actionnement. Avantageusement, l'invention s'applique en particulier à un inhalateur dans lequel ladite roue d'indexage est déplaçable non seulement en rotation, mais aussi en direction de moyens de perçage 40 qui restent fixes par rapport au corps de l'inhalateur. Bien entendu, d'autres applications sont envisageables sans sortir du cadre de la présente invention tel que défini par les revendications annexées.

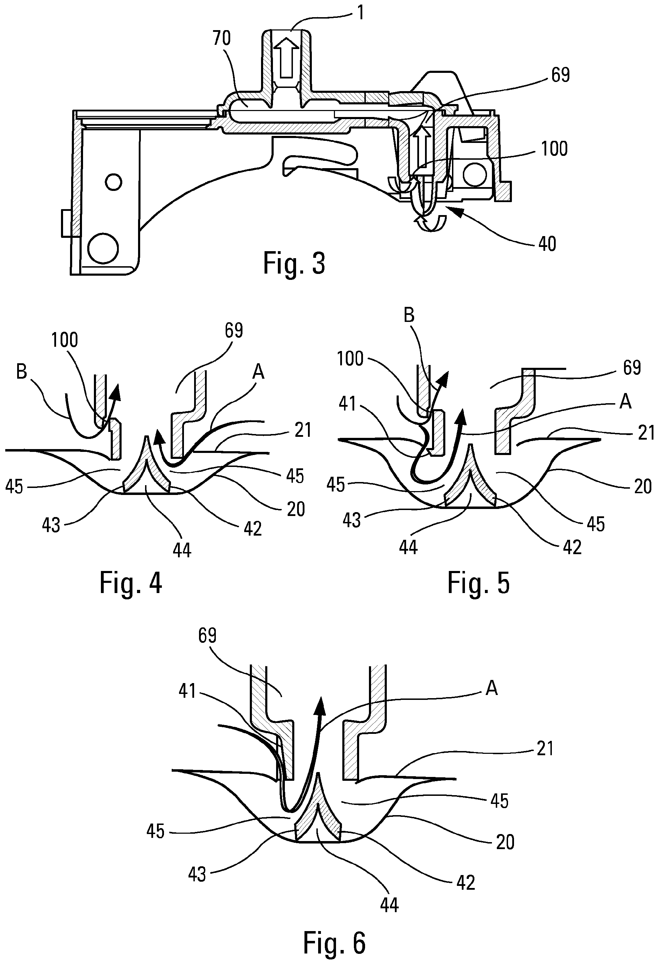

Le dispositif d'ouverture comporte des moyens de perçage (ou éléments de perçage) 40 adaptés à percer la paroi de fermeture 21 d'un blister 20 à chaque actionnement. Cet élément de perçage 40 est creux et définit un canal d'expulsion 69. Il comporte au moins une extrémité de perçage, de préférence deux extrémités de perçage 42, 43. Ces extrémités de perçage percent la paroi de fermeture 21 et pénètrent à l'intérieur du blister. Chaque extrémité de perçage 42, 43 comporte une ouverture 45 permettant à la poudre de sortir du blister pour être transportée dans le canal d'expulsion. De préférence, le dispositif d'ouverture est déclenché par l'inhalation de l'utilisateur, et l'écoulement d'inhalation pénètre dans le blister ouvert, se mélange à la poudre et l'entraîne vers le canal d'expulsion à travers la ou les ouverture(s) 45 de la ou des extrémité(s) de perçage 42, 43. De préférence, l'écoulement d'inhalation pénètre à l'intérieur du blister ouvert en passant à l'extérieur de l'élément de perçage 40, comme représenté par la flèche A sur les figures 4 à 6.

Selon l'invention, le dispositif d'ouverture comporte au moins un canal d'air 100 également relié au canal d'expulsion 69 et à travers lequel s'écoule un écoulement d'air (flèche B) qui lors de l'inhalation va se mélanger dans le canal d'expulsion 69 avec l'écoulement d'air et de poudre (flèche A) sortant du blister 20 et passant à l'intérieur de l'élément de perçage creux 40. Cet écoulement d'air favorise donc un transport efficace et fiable de la poudre expulsée du blister vers l'orifice de distribution 1 de l'inhalateur. Il permet aussi de réduire la variabilité de la dose émise. En effet, ces canaux d'air 100 formés dans l'élément de perçage 40 ont des géométries d'éléments moulés industriellement reproductibles, permettant de stabiliser les performances et les paramètres fluidiques. En particulier, l'écoulement B (géométriquement maîtrisable de manière industrielle) peut être suffisamment prépondérant par rapport au flux principal A (plus difficilement maîtrisable) pour diminuer la variabilité globale des performances.

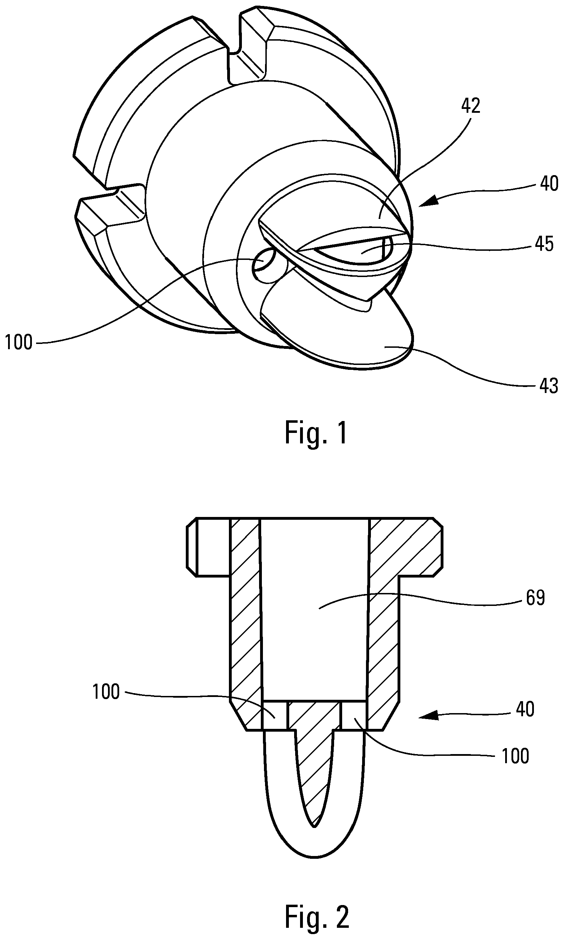

Ce canal d'air 100 peut être rectiligne, parallèle audit canal d'expulsion 69 et de section transversale constante, comme représenté sur les figures 1 à 3. En variante, il pourrait être de forme quelconque, de section variable, par exemple tronconique, en fonction des effets souhaités au moment où il se mélange à l'écoulement d'air et de poudre dans le canal d'expulsion 69.

Selon l'invention, ledit au moins un canal d'air 100 est réalisé sur une paroi de l'élément de perçage 40 qui ne pénètre pas dans le blister 20, et qui est sensiblement perpendiculaire au canal d'expulsion 69, comme représenté sur les figures 1 à 3. Chaque canal d'air 100 est ainsi réalisé par un trou traversant réalisé dans une paroi de l'élément de perçage 40, ledit trou traversant étant orienté sensiblement parallèlement audit canal d'expulsion. Cette mise en oeuvre permet d'obtenir un effet maximal avec l'écoulement d'air orienté dans le même sens que l'écoulement d'air et de poudre.

Les moyens de perçage 40 ont de préférence une forme appropriée de telle sorte que les parties de parois découpées du blister 20 se plient vers l'intérieur du blister sans recouvrir les ouvertures formées par ces moyens de perçage 40. Avantageusement, ces moyens de perçage 40 comportent au moins deux extrémités de perçage opposées 42, 43 qui sont séparées l'une de l'autre par un écart approprié 44. Ces moyens de perçage 40 créent avantageusement un pliage central avec des parties de parois découpées permettant aux ouvertures formées de ne pas du tout être recouvertes par une quelconque partie de parois découpée. Avantageusement, comme représenté sur la figure 1, chaque extrémité de perçage 42, 43 est en forme de coupelle, lesdites coupelles étant disposées dos à dos et ayant des bords coupants. Avantageusement, l'air entrant (flèche A) pénètre dans le blister ouvert ou passant à l'extérieur desdits moyens de perçage 40. Ceci peut par exemple être obtenu grâce au fait que les blisters 20 se déplacent vers leur position d'ouverture selon une ligne courbe. Ce déplacement selon une courbe provoque une ou des ouverture(s) légèrement plus large(s) que les dimensions externes de l'élément de perçage 40. Ceci permet donc à de l'air d'inhalation de s'écouler à l'extérieur dudit élément de perçage 40 pour pénétrer à l'intérieur du blister. Si nécessaire, des moyens spécifiques pour réaliser une ouverture à l'extérieur dudit élément de perçage 40 pourraient également être prévus, tels que par exemple des nervures 41 ou tout autre profil externe approprié sur ledit élément de perçage 40 (voir figure 6). De préférence, l'écoulement d'air sortant chargé avec la poudre (flèche A) sort du blister 21 en passant à l'intérieur desdits moyens de perçage creux 40, comme représenté sur les figures 3 à 6. La forme spécifique de l'élément de 40 tel que représentée sur les figures fournit une découpe du type « Louvre » qui fournit tous les avantages susmentionnés, et en particulier qui évite que les trous créés par le perçage ne soient obturés même partiellement par des parties de parois percées. Ceci permet d'assurer un vidage le plus complet possible du blister, et fournit donc une efficacité maximale pour le distributeur. De même, la reproductibilité du dosage est optimale, la même quantité de poudre étant à chaque fois expulsée grâce au dispositif de l'invention. De préférence, l'élément de perçage 40 comporte deux canaux d'air 100 disposés symétriquement entre les deux extrémités de perçage 42, 43, comme représenté sur les figures 1 à 3. Bien entendu, un nombre quelconque de canaux d'air 100, qui pourraient être de dimensions différentes les uns des autres, est aussi envisageable.

Lorsque le réservoir 20 se déplace vers sa position d'ouverture pour être ouvert par les moyens de perçage, ces moyens de perçage 40 sont fixes par rapport au corps de l'inhalateur. Toutefois, selon un exemple particulier qui ne tombe pas sous l'objet de la présente invention tel que défini par les revendications annexées, il est envisageable que ces moyens de perçage puissent également être mobiles pendant la phase d'ouverture du réservoir. Par exemple, les moyens de perçage pourraient se déplacer en direction du réservoir pendant que le réservoir se déplace en direction des moyens de perçage. Dans une autre variante qui ne tombe pas non plus sous l'objet de la présente invention, on pourrait également envisager que le réservoir et les moyens de perçage se déplacent dans la même direction lors de l'actionnement, le réservoir se déplaçant plus rapidement dans cette direction de sorte qu'il vient en contact avec lesdits moyens de perçage pour être ouvert.

L'inhalateur comporte en outre une chambre de distribution 70 qui est destinée à recevoir la dose de poudre après ouverture d'un réservoir respectif. Avantageusement, cette chambre de distribution 70 est pourvue d'au moins une bille 75 (non représentée) qui se déplace à l'intérieur de ladite chambre 70 pendant l'inhalation, pour améliorer la distribution du mélange air et poudre après ouverture d'un réservoir, afin d'augmenter l'efficacité du dispositif.

Comme représenté sur les dessins, il peut être avantageux que les moyens d'ouverture, en particulier les moyens de perçage 40 soient formés directement sur ladite chambre de distribution 70, par exemple à l'extrémité d'un canal 69, formant canal d'expulsion, menant à ladite chambre 70.

Les réservoirs individuels ou blisters 20 sont formés sur une bande allongée (non représentée), qui peut être stockée sous forme de bobine à l'intérieur du corps du dispositif d'inhalation. La bande de blister peut être déplacée par l'utilisateur avantageusement au moyen de la roue de guidage (non représentée) qui comporte avantageusement au moins un, de préférence plusieurs évidements dont la forme correspond sensiblement à celle des blisters. Ainsi, lorsque cette roue de guidage tourne, elle entraîne la bande de blister. Bien entendu en variante ou de manière additionnelle, on pourrait utiliser d'autres moyens d'avancée de bande de blisters, par exemple en prévoyant un profil sur les bords longitudinaux latéraux de la bande de blister ledit profil étant adapté à coopérer avec des moyens d'entraînement appropriés. De même, des trous ménagés le long des bords latéraux de la bande de blister pourraient également être utilisés pour faire avancer la bande de blister au moyen de roues dentées coopérant avec lesdits trous.

Diverses modifications sont également possibles pour un homme du métier sans sortir du cadre de la présente invention tel que défini par les revendications annexées.

## Revendications

1. Dispositif d'inhalation comportant une pluralité de réservoirs individuels réalisée sous la forme d'une bande allongée comportant plusieurs réservoirs individuels (20) disposés les uns à la suite des autres, ledit dispositif comportant un dispositif d'ouverture pour ouvrir un réservoir individuel (20) contenant une dose unique de poudre, ledit réservoir (20) comportant une paroi de fermeture (21), ledit dispositif d'ouverture comportant des moyens de perçage creux (40) adaptés à percer ladite paroi de fermeture (21) du réservoir (20), lesdits moyens de perçage creux (40) étant reliés à un canal d'expulsion (69) pour transporter la poudre en direction d'un orifice de distribution (1) dudit dispositif d'inhalation, ledit dispositif d'ouverture comportant au moins un canal d'air (100) relié audit canal d'expulsion (69), de sorte que lors de l'inhalation, un écoulement d'air (B) passant à travers ledit au moins un canal d'air (100) se mélange, dans ledit canal d'expulsion (69), à l'écoulement d'air et de poudre (A) sortant du réservoir (20) à travers lesdits moyens de perçage creux (40), ledit dispositif d'ouverture étant fixe par rapport au corps du dispositif d'inhalation pendant l'ouverture du réservoir (20), **caractérisé en ce que** ledit au moins un canal d'air (100) est formé par un trou traversant réalisé dans une paroi desdits moyens de perçage qui ne pénètre pas à l'intérieur du réservoir (20) et qui est sensiblement perpendiculaire au canal d'expulsion, ledit au moins un trou traversant étant orienté sensiblement parallèlement audit canal d'expulsion (69).

2. Dispositif selon la revendication 1, dans lequel ledit au moins un canal d'air (100) est sensiblement rectiligne.

3. Dispositif selon l'une quelconque des revendications précédentes, dans lequel ledit au moins un canal d'air (100) a une section sensiblement constante sur sa longueur.

4. Dispositif selon la revendication 1 ou 2, dans lequel ledit au moins un canal d'air (100) a une section variable sur sa longueur.

5. Dispositif selon l'une quelconque des revendications précédentes, dans lequel lesdits moyens de perçage (40) comportent deux extrémités de perçage opposées (42, 43) séparées par un écart (44).

6. Dispositif selon la revendication 5, dans lequel lesdits moyens de perçage (40) comportent deux canaux d'air (100) disposés symétriquement entre lesdites deux extrémités de perçage (42, 43).

7. Dispositif selon l'une quelconque des revendications précédentes, dans lequel l'écoulement d'air entrant pénètre dans le réservoir ouvert (20) en passant à l'extérieur desdits moyens de perçage (40).

8. Dispositif selon la revendication 7, dans lequel lesdits moyens de perçage (40) comportent au moins une projection externe (41), telle qu'une nervure, pour former au moins un passage pour l'air entrant dans le réservoir ouvert (20).

9. Dispositif selon l'une quelconque des revendications précédentes, dans lequel ledit dispositif d'ouverture est activé par l'inhalation de l'utilisateur.

10. Dispositif selon la revendication 9, dans lequel, lors d'une inhalation, un réservoir (20) est déplacé contre ledit dispositif d'ouverture, de telle sorte que ledit réservoir est ouvert pendant qu'un premier écoulement d'inhalation (A) entre dans le réservoir en passant à l'extérieur desdits moyens de perçage (40), réalisant l'expulsion du produit fluide vers le canal d'expulsion (69) en passant à l'intérieur desdits moyens de perçage (40), ledit premier écoulement d'inhalation (A) se mélangeant dans le canal d'expulsion (69) avec un second écoulement d'inhalation (B) passant à travers ledit au moins un canal d'air (100).

## Patentansprüche

1. Inhalationsvorrichtung, umfassend eine Vielzahl von einzelnen Behältern, die in Form eines langgestreckten Streifens gebildet sind, der mehrere einzelne Behälter (20) umfasst, die hintereinander angeordnet sind, wobei die Vorrichtung eine Öffnungsvorrichtung umfasst, um einen einzelnen Behälter (20) zu öffnen, der eine einzige Pulverdosis enthält, wobei der Behälter (20) eine Verschlusswand (21) umfasst, wobei die Öffnungsvorrichtung hohle Durchstechmittel (40) umfasst, die dazu geeignet sind, die Verschlusswand (21) des Behälters (20) zu durchstechen, wobei die hohlen Durchstechmittel (40) mit einem Austreibkanal (69) verbunden sind, um das Pulver in Richtung zu einer Ausgabeöffnung (1) der Inhalationsvorrichtung zu transportieren, wobei die Öffnungsvorrichtung mindestens einen Luftkanal (100) umfasst, der mit dem Austreibkanal (69) verbunden ist, so dass bei der Inhalation ein Luftstrom (B), der durch den mindestens einen Luftkanal (100) tritt, sich in dem Austreibkanal (69) mit dem Luft- und Pulverstrom (A), der durch die hohlen Durchstechmittel (40) aus dem Behälter (20) austritt, vermischt, wobei die Öffnungsvorrichtung in Bezug auf den Körper der Inhalationsvorrichtung während der Öffnung des Behälters (20) feststehend ist, **dadurch gekennzeichnet, dass** der mindestens eine Luftkanal (100) durch ein Durchgangsloch gebildet ist, welches in einer Wand der Durchstechmittel gebildet ist, die nicht in das Innere des Behälters (20) eindringt und die im Wesentlichen senkrecht zum Austreibkanal liegt, wobei das mindestens eine Durchgangsloch im Wesentlichen parallel zum Austreibkanal (69) ausgerichtet ist.

2. Vorrichtung nach Anspruch 1, wobei der mindestens eine Luftkanal (100) im Wesentlichen geradlinig ist.

3. Vorrichtung nach einem der vorhergehenden Ansprüche, wobei der mindestens eine Luftkanal (100) einen über seine Länge im Wesentlichen konstanten Querschnitt hat.

4. Vorrichtung nach Anspruch 1 oder 2, wobei der mindestens eine Luftkanal (100) einen über seine Länge variablen Querschnitt hat.

5. Vorrichtung nach einem der vorhergehenden Ansprüche, wobei die Durchstechmittel (40) zwei gegenüberliegende Durchstechenden (42, 43) umfassen, die durch einen Abstand (44) voneinander getrennt sind.

6. Vorrichtung nach Anspruch 5, wobei die Durchstechmittel (40) zwei Luftkanäle (100) umfassen, die symmetrisch zwischen den beiden Durchstechenden (42, 43) angeordnet sind.

7. Vorrichtung nach einem der vorhergehenden Ansprüche, wobei der eintretende Luftstrom in den offenen Behälter (20) eindringt, indem er aus den Durchstechmitteln (40) nach außen tritt.

8. Vorrichtung nach Anspruch 7, wobei die Durchstechmittel (40) mindestens einen äußeren Vorsprung (41), wie eine Rippe, umfassen, um mindestens einen Durchgang für die in den offenen Behälter (20) eintretende Luft zu bilden.

9. Vorrichtung nach einem der vorhergehenden Ansprüche, wobei die Öffnungsvorrichtung durch Inhalation des Benutzers aktiviert wird.

10. Vorrichtung nach Anspruch 9, wobei bei einer Inhalation ein Behälter (20) gegen die Öffnungsvorrichtung verlagert wird, so dass der Behälter geöffnet wird, während ein erster Inhalationsstrom (A) in den Behälter eintritt, indem er aus den Durchstechmitteln (40) nach außen tritt, wodurch das Austreiben des fluiden Produkts zum Austreibkanal (69) bewirkt wird, indem dieses ins Innere der Durchstechmittel (40) fließt, wobei sich der erste Inhalationsstrom (A) in dem Austreibkanal (69) mit einem zweiten Inhalationsstrom (B) vermischt, der durch den mindestens einen Luftkanal (100) hindurchgeht.

## Claims

1. An inhaler device comprising a plurality of individual reservoirs made in the form of an elongate strip comprising a plurality of individual reservoirs (20) disposed one behind another, said device including an opening device for opening an individual reservoir (20) containing a single dose of powder, said reservoir (20) including a closure wall (21), said opening device comprising hollow perforator means (40) that are adapted to perforate said closure wall (21) of the reservoir (20), said hollow perforator means (40) being connected to an expulsion channel (69) so as to transport the powder towards a dispenser orifice (1) of said inhaler, said opening device including at least one air channel (100) connected to said expulsion channel (69), such that during inhalation, an air flow (B) passing through said at least one air channel (100) mixes, in said expulsion channel (69), with the air and powder flow (A) leaving the reservoir (20) through said hollow perforator means (40), said opening device doing not move relative to the body of the inhaler while the reservoir (20) is being opened, said inhaler device being **characterized in that** said at least one air channel (100) is formed by a through hole made in a wall of said perforator means that does not penetrate into the reservoir (20) and that is substantially perpendicular to the expulsion channel, said at least one through hole being oriented substantially parallel to said expulsion channel (69).

2. A device according to claim 1, wherein said at least one air channel (100) is substantially rectilinear.

3. A device according to any preceding claim, wherein said at least one air channel (100) has a section that is substantially constant along its length.

4. A device according to any one of claims 1 or 2, wherein said at least one air channel (100) has a section that varies along its length.

5. A device according to any preceding claim, wherein said perforator means (40) include two opposite perforator ends (42, 43) that are spaced apart by a distance (44).

6. A device according to claim 5, wherein said perforator means (40) include two air channels (100) that are disposed symmetrically between said two perforator ends (42, 43).

7. A device according to any preceding claim, wherein the flow of incoming air penetrates into the open reservoir (20) by passing over the outside of said perforator means (40).

8. A device according to claim 7, wherein said perforator means (40) include at least one external projection (41), such as a spline, so as to form at least one passage for the incoming air entering into the open reservoir (20).

9. A device according to any preceding claim, wherein said opening device is activated by the user inhaling.

10. A device according to claim 11, wherein during inhalation, a reservoir (20) is moved against said opening device, such that said reservoir is opened while a first inhalation flow (A) enters into the reservoir by passing over the outside of said perforator means (40), causing the fluid to be expelled towards the expulsion channel (69) by passing into said perforator means (40), said first inhalation flow (A) mixing, in the expulsion channel (69), with a second inhalation flow (B) passing through said at least one air channel (100).
